# EUROPÄISCHE PATENTSCHRIFT

(11) **EP 0 863 706 B1**
(45) Veröffentlichungstag und Bekanntmachung des Hinweises auf die Patenterteilung: **20.02.2002**
(21) Anmeldenummer: 96938069.0
(22) Anmeldetag: 04.11.1996
(51) Int. Cl.: A01N 47/36, C07D 239/52

(54) **VERFAHREN ZUR BEKÄMPFUNG VON UNERWÜNSCHTEM PFLANZENWUCHS IN REISKULTUREN**
METHOD OF CONTROLLING UNDESIRED PLANT GROWTH IN RICE
PROCEDE POUR LUTTER CONTRE LA CROISSANCE INDESIREE DE PLANTES DANS LES CULTURES DE RIZ

(30) Priorität: 23.11.1995 DE 19543648
(43) Veröffentlichungstag der Anmeldung: 16.09.1998
(73) Patentinhaber: Aventis CropScience GmbH, 65929 Frankfurt am Main (DE)
(72) Erfinder: SCHNABEL, Gerhard, D-63868 Grosswallstadt (DE); WILLMS, Lothar, D-65719 Hofheim (DE); BAUER, Klaus, D-63456 Hanau (DE)
(86) Internationale Anmeldenummer: EP9604798
(87) Internationale Veröffentlichungsnummer: WO9718711

(56) Entgegenhaltungen:
- EP-A- 0 084 020
- EP-A- 0 116 518
- US-A- 4 369 058

## Beschreibung

Die Erfindung liegt auf dem Gebiet des Pflanzenschutzes, insbesondere des Einsatzes von Pflanzenschutzmitteln gegen monokotyle und dikotyle Unkräuter in Pflanzenkulturen.

EP-A-116518 offenbart N-Phenylsulfonyl-N'-pyrimidinyl- oder N'-triazinyl-harnstoffe der Formel (I), die gute pre- und postemergent selektive herbizide und wuchsregulierende Eigenschaften aufweisen. EP-A-84020 offenbart herbizid wirksame und pflanzenwuchsregulierende N-Arylsulfonyl-N'-pyrimidinylharnstoffe der Formel (I), die selektiv in Nutzpflanzen-kulturen wirken. Die in EP-A-84020 und EP-A-116518 offenbarten Verbindungen eignen sich zum Einsatz in Reiskulturen.

Aus US-A-4,369,058, US-A-4,453,971 und US-A-4,225,337 sind N-acylierte 2-Aminophenylsulfonylharnstoffe bekannt, die als Mittel zur Bekämpfung eines breiten Spektrums mono- und dikotyler Unkräuter beschrieben sind. Dabei werden die beschriebenen Sulfonylharnstoffe als Herbizide klassifiziert, die eine breite Wirkung gegen unerwünschten Pflanzenwuchs bei einer Aufwandmenge zwischen 0,1 und 20 kg Aktivsubstanz pro Hektar (kg a.i./ha), vorzugsweise zwischen 0,2 kg a.i./ha und 10 kg a.i./ha, entfalten. Nach den dort beschriebenen Tests zeigen die getesteten Verbindungen meist keine ausreichende Selektivität in Nutzkulturen, d.h. die Verbindungen verursachen erhebliche Schäden an den Nutzpflanzen oder besitzen keine oder nur geringe herbizide Wirkung gegen Schadpflanzen. Insbesondere weisen die herbizid wirksamen Verbindungen nach den beschriebenen Tests in Reiskulturen auch erhebliche Schädigungen bei den Reispflanzen auf.

Es wurde nun überraschenderweise gefunden, daß bestimmte Sulfonylharnstoffherbizide eine ausgezeichnete Selektivität im Reis aufweisen, wobei überraschenderweise auch bei niedrigen Wirkstoffdosierungen eine hervorragende Wirkung gegen die in Reiskulturen typisch auftretenden Schadpflanzen erhalten bleibt. Insbesondere werden im Reisanbau vorkommende, oft schwer bekämpfbare perennierende Unkräuter, beispielsweise Sagittaria spec., Cyperus serotinus, Scirpus maritimus, Eleocharis spec. und Scirpus juncoides, sowie ein breites Spektrum an annuellen Unkräutern wirksam bekämpft. Die betreffenden Sulfonylharnstoffherbizide eignen sich deshalb besonders zur Bekämpfung von Schadpflanzen in Reiskulturen.

Gegenstand der vorliegenden Erfindung ist somit ein Verfahren zur Bekämpfung von Schadpflanzen in Reiskulturen, dadurch gekennzeichnet, daß man eine oder mehrere Verbindungen der Formel (I) oder deren Salze, 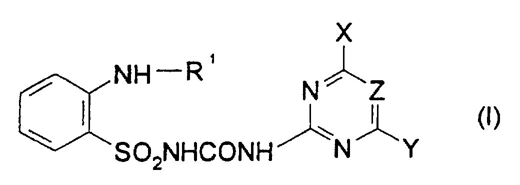 worin
- R¹: einen Acylrest aus der Gruppe CO-R², CS-R², CO-OR³, CS-OR³, CO-SR⁴, CS-SR⁴, CO-NR⁵R⁶ und CS-NR⁵R⁶,
- R²: H, (C₁-C₆)Alkyl, (C₃-C₆)Cycloalkyl, (C₃-C₆)Cycloalkyl-(C₁-C₄)alkyl, (C₂-C₆)Alkenyl oder (C₂-C₆)Alkinyl, wobei jeder der 5 letztgenannten Reste unsubstituiert oder durch einen oder mehrere Reste aus der Gruppe Halogen, (C₁-C₄)Alkoxy, (C₁-C₄)Haloalkoxy, (C₁-C₄)Alkylthio, (C₁-C₄)Haloalkylthio, CN, NH₂, Mono- und Di(C₁-C₄)alkylamino und im Falle cyclischer Reste auch (C₁-C₄)Alkyl und (C₁-C₄)Haloalkyl substituiert ist,
- R³: einen Rest aus der Gruppe der für R² möglichen Reste, außer H,
- R⁴: einen Rest aus der Gruppe der für R³ möglichen Reste,
- R⁵,R⁶: unabhängig voneinander jeweils einen Rest aus der Gruppe der für R² möglichen Reste oder die Gruppe
- NR⁵R⁶: gemeinsam einen am N-Atom gebundenen substituierten oder unsubstituierten 4-, 5- oder 6-gliedrigen gesättigten oder ungesättigten heterocyclischen Rest, der neben dem N-Atom kein weiteres Heteroringatom oder weitere Heteroringatome enthält, vorzugsweise keines oder 1 oder 2 Heteroringatome aus der Gruppe N, O und S enthält, der unsubstituiert oder durch einen oder mehrere Reste aus der Gruppe (C₁-C₄)Alkyl, (C₁-C₄)Haloalkyl, Halogen, (C₁-C₄)Alkoxy und (C₁-C₄)Haloalkoxy substituiert ist,
- X,Y: unabhängig voneinander Halogen, (C₁-C₆)Alkyl, (C₁-C₆)Alkoxy, (C₁-C₆)Alkylthio, wobei jeder der drei letztgenannten Reste unsubstituiert oder durch einen oder mehrere Reste aus der Gruppe Halogen, (C₁-C₄)Alkoxy und (C₁-C₄)Alkylthio substituiert ist, oder (C₃-C₆)Cycloalkyl, (C₃-C₆)Alkenyl, (C₃-C₆)Alkinyl, (C₃-C₆)Alkenyloxy oder (C₃-C₆)Alkinyloxy und
- Z: CH oder N
bedeuten,
auf die Anbaufläche, welche Schad- und Reispflanzen oder deren Samen enthält, in einer wirksamen Menge von 0,0001 bis 0,5 kg a.i./ha, vorzugsweise 0,001 bis 0,2 kg a.i./ha, insbesondere 0,005 bis 0,12 kg a.i./ha, appliziert.

Von besonderem Interesse sind erfindungsgemäße Verfahren mit Verbindungen der Formel (I) oder deren Salzen, worin
- R¹: CO-R², CS-R², CO-OR³, CS-OR³, CO-SR⁴_{,} CS-SR⁴, CO-NR⁵R⁶ und CS-NR⁵R⁶, vorzugsweise CO-R², CO-OR³ oder CO-NR⁵R⁶,
- R²: H, (C₁-C₆)Alkyl, (C₃-C₆)Cycloalkyl, (C₃-C₆)Cycloalkyl-(C₁-C₂)alkyl, (C₂-C₄)Alkenyl oder (C₂-C₄)Alkinyl, wobei jeder der 5 letztgenannten Reste unsubstituiert oder durch einen oder mehrere Reste aus der Gruppe Halogen, (C₁-C₃)Alkoxy, (C₁-C₃)Haloalkoxy, (C₁-C₃)Alkylthio, (C₁-C₃)Haloalkylthio, CN, NH₂, Mono- und Di(C₁-C₃)alkylamino und im Falle cyclischer Reste auch (C₁-C₄)Alkyl und (C₁-C₄)Haloalkyl substituiert ist,
- R³: einen Rest aus der Gruppe der für R² möglichen Reste, außer H,
- R⁴: einen Rest aus der Gruppe der für R³ möglichen Reste,
- R⁵,R⁶: unabhängig voneinander jeweils einen Rest aus der Gruppe der für R² möglichen Reste oder die Gruppe
- NR⁵R⁶: gemeinsam einen am N-Atom gebundenen substituierten oder unsubstituierten 4-, 5- oder 6-gliedrigen gesättigten heterocyclischen Rest der Formel (A-1), (A-2), (A-3) oder (A-4) 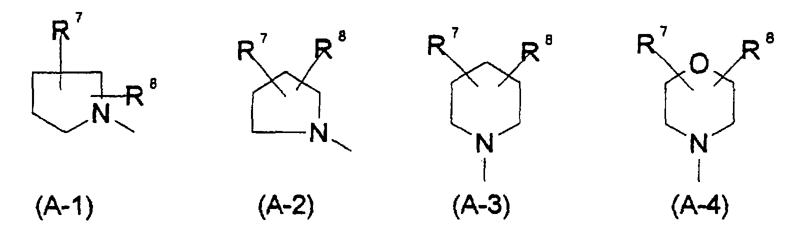
- R⁷,R⁸: unabhängig voneinander H, Halogen, (C₁-C₃)Alkyl, (C₁-C₃)Haloalkyl, (C₁-C₃)Alkoxy oder (C₁-C₃)Haloalkoxy,
- X,Y: unabhängig voneinander Halogen, (C₁-C₄)Alkyl, (C₁-C₄)Alkoxy, (C₁-C₄)Alkylthio, wobei jeder der drei letztgenannten Reste unsubstituiert oder durch einen oder mehrere Reste aus der Gruppe Halogen, (C₁-C₄)Alkoxy und (C₁-C₄)Alkylthio substituiert ist, oder (C₃-C₆)Cycloalkyl, (C₃-C₄)Alkenyl, (C₃-C₄)Alkinyl, (C₃-C₄)Alkenoxy oder (C₃-C₄)Alkinoxy, und
- Z: CH oder N bedeuten.

Bevorzugt sind erfindungsgemäße Verfahren mit Verbindungen der Formel (I) oder deren Salzen, worin
- R²: H, (C₁-C₄)Alkyl, (C₁-C₄)Haloalkyl, (C₁-C₃)Alkoxy-(C₁-C₄)alkyl, (C₁-C₃)Alkylthio-(C₁-C₄)alkyl, (C₃-C₆)Cycloalkyl, (C₃-C₆)Cycloalkylmethyl, vorzugsweise H, (C₁-C₄)Alkyl oder (C₃-C₆)Cycloalkyl, insbesondere C₂H₅, i-C₃H₇ oder Cyclopropyl,
- R³: (C₁-C₄)Alkyl, (C₁-C₄)Haloalkyl, (C₁-C₃)Alkoxy-(C₁-C₄)alkyl, (C₁-C₃)Alkylthio-(C₁-C₄)alkyl, (C₃-C₆)Cycloalkyl, (C₃-C₆)Cycloalkylmethyl, (C₃-C₄)Alkenyl oder (C₃-C₄)Alkinyl, vorzugsweise (C₁-C₄)Alkyl oder (C₃-C₆)Cycloalkyl, insbesondere Methyl oder Ethyl,
- R⁴: einen Rest aus der Gruppe der für R³ möglichen Reste, vorzugsweise (C₁-C₄)Alkyl,
- R⁵,R⁶: unabhängig voneinander H oder (C₁-C₄)Alkyl,
einer der Reste X und Y (C₁-C₄)Alkyl, (C₁-C₄)Alkoxy, (C₁-C₄)Haloalkyl oder (C₁-C₄)Haloalkoxy, vorzugsweise Methyl, Methoxy oder Ethoxy, insbesondere Methoxy,
und der andere der Reste X und Y Halogen, (C₁-C₄)Alkyl, (C₁-C₄)Alkoxy, (C₁-C₄)Alkylthio, wobei die drei letztgenannten Reste unsubstituiert oder durch einen oder mehrere Reste aus der Gruppe Halogen, (C₁-C₄)Alkoxy und (C₁-C₄)Alkylthio substituiert sind, oder (C₃-C₆)Cycloalkyl, (C₂-C₄)Alkenyl, (C₂-C₄)Alkinyl, (C₃-C₄)Alkenoxy oder (C₃-C₄)Alkinoxy, vorzugsweise Methyl, Methoxy, Ethoxy, Chlor, Fluor oder CF₃, insbesondere Methoxy, und
- Z: CH oder N , insbesondere CH,
bedeuten.

Besonders bevorzugt sind erfindungsgemäße Verfahren mit Verbindungen der genannten Formel (I) oder deren Salze, worin
R¹ CO-R² oder CO-OR³, beispielsweise COOCH₃, COOC₂H₅, COO-n-C₃H₇, COO-i-C₃H₇, COO-cyclo-C₃H₅, CO-C₂H₅, CO-n-C₃H₅, COO-i-C₃H₇ oder CO-cyclo-C₃H₇ bedeutet.

In der Formel (I) und den im folgenden verwendeten Formeln können die Reste Alkyl, Alkoxy, Haloalkyl, Haloalkoxy, Alkylamino und Alkylthio sowie die entsprechenden ungesättigten und/oder substituierten Reste im Kohlenstoffgerüst jeweils geradkettig oder verzweigt sein. Wenn nicht speziell angegeben, sind bei diesen Resten die niederen Kohlenstoffgerüste, z. B. mit 1 bis 4 C-Atomen bzw. bei ungesättigten Gruppen mit 2 bis 4 C-Atomen, bevorzugt. Alkylreste, auch in den zusammengesetzten Bedeutungen wie Alkoxy, Haloalkyl usw., bedeuten z. B. Methyl, Ethyl, n- oder i-Propyl, n-, i-, t- oder 2-Butyl, Pentyle, Hexyle, wie n-Hexyl, i-Hexyl und 1,3-Dimethylbutyl, Heptyle, wie n-Heptyl, 1-Methylhexyl und 1,4-Dimethylpentyl; Alkenyl- und Alkinylreste haben die Bedeutung der den Alkylresten entsprechenden möglichen ungesättigten Reste, Alkenyl bedeutet z.B. Allyl, 1-Methylprop-2-en-1-yl, 2-Methyl-prop-2-en-1-yl, But-2-en-1-yl, But-3-en-1-yl, 1-Methyl-but-3-en-1-yl und 1-Methyl-but-2-en-1-yl; Alkinyl bedeutet z.B. Propargyl, But-2-in-1-yl, But-3-in-1-yl, 1-Methyl-but-3-in-1-yl.
Alkenyl in der Form "(C₃-C₄)Alkenyl" oder "(C₃-C₆)Alkenyl" bedeutet vorzugsweise einen Alkenylrest mit 3 bis 4 bzw. 3 bis 6 C-Atomen, bei dem die Doppelbindung nicht zwischen C-1 und C-2 liegt (C-1 bezeichnet dabei die Position mit "yl"); entsprechendes gilt für (C₃-C₆)Alkinyl bzw. (C₃-C₆)Alkinyl.

Halogen bedeutet beispielsweise Fluor, Chlor, Brom oder lod. Haloalkyl, -alkenyl und -alkinyl bedeuten durch Halogen, vorzugsweise durch Fluor, Chlor und/oder Brom, insbesondere durch Fluor oder Chlor, teilweise oder vollständig substituiertes Alkyl, Alkenyl bzw. Alkinyl, z.B. CF₃, CHF₂, CH₂F, CF₃CF₂, CH₂FCHCl, CCl₃, CHCl₂, CH₂CH₂Cl; Haloalkoxy ist z.B. OCF₃, OCHF₂, OCH₂F, CF₃CF₂O, OCH₂CF₃ und OCH₂CH₂Cl; entsprechendes gilt für Haloalkenyl und andere durch Halogen substituierte Reste.

Ein heterocyclischer Rest oder Ring kann gesättigt, ungesättigt oder heteroaromatisch sein; er enthält ein oder mehrere Heteroringatome, vorzugsweise aus der Gruppe N, O und S; vorzugsweise ist er 5- oder 6-gliedrig und enthält 1, 2 oder 3 Heteroringatome. Der Rest kann z.B. ein heteroaromatischer Ring wie Pyridyl, Pyrimidinyl, Pyridazinyl, Pyrazinyl, Thienyl, Thiazolyl, Oxazolyl, Furyl, Pyrrolyl, Pyrazolyl und Imidazolyl, inklusive bicyclische oder polycyclische aromatische oder araliphatische Verbindungen, z.B. Chinolinyl, Benzoxazolyl etc. sein oder auch ein partiell oder ganz hydrierter Rest wie Oxiranyl, Pyrrolidyl, Piperidyl, Piperazinyl, Dioxolanyl, Morpholinyl, Tetrahydrofuryl.
Als Substituenten für einen substituierten heterocyclischen Rest kommen die weiter unten genannten Substituenten in Frage, zusätzlich auch Oxo. Die Oxogruppe kann auch an den Heteroringatomen, die in verschiedenen Oxidationsstufen existieren können, z.B. bei N und S, auftreten.

Substituierte Reste, wie substituierte Kohlenwasserstoffreste, z.B. substituiertes Alkyl, Alkenyl, Alkinyl, Aryl, Phenyl und Benzyl, oder substituiertes Heteroaryl, ein substituierter bicyclischer Rest oder Ring oder ein substituierter bicyclischer Rest, gegebenenfalls mit aromatischen Anteilen, bedeuten beispielsweise einen vom unsubstituierten Grundkörper abgeleiteten substituierten Rest, wobei die Substituenten beispielsweise einen oder mehrere, vorzugsweise 1, 2 oder 3 Reste aus der Gruppe Halogen, Alkoxy, Haloalkoxy, Alkylthio, Hydroxy, Amino, Nitro, Cyano, Azido, Alkoxycarbonyl, Alkylcarbonyl, Formyl, Carbamoyl, Mono- und Dialkylaminocarbonyl, substituiertes Amino wie Acylamino, Mono- und Dialkylamino, und Alkylsulfinyl, Haloalkylsulfinyl, Alkylsulfonyl, Haloalkylsulfonyl und, im Falle cyclischer Reste, auch Alkyl und Haloalkyl sowie den genannten gesättigten kohlenwasserstoffhaltigen Resten entsprechende ungesättigte aliphatische Reste, wie Alkenyl, Alkinyl, Alkenyloxy, Alkinyloxy etc. bedeuten. Bei Resten mit C-Atomen sind solche mit 1 bis 4 C-Atomen, insbesondere 1 oder 2 C-Atomen, bevorzugt. Bevorzugt sind in der Regel Substituenten aus der Gruppe Halogen, z.B. Fluor und Chlor, C₁-C₄-Alkyl, vorzugsweise Methyl oder Ethyl, C₁-C₄-Haloalkyl, vorzugsweise Trifluormethyl, C₁-C₄-Alkoxy, vorzugsweise Methoxy oder Ethoxy, C₁-C₄-Haloalkoxy, Nitro und Cyano. Besonders bevorzugt sind dabei die Substituenten Methyl, Methoxy und Chlor.

Von der Formel (I) werden auch einzelne Stereoisomere und deren Gemische umfaßt. Solche Stereoisomere enthalten ein oder mehrere asymmetrische C-Atome oder auch Doppelbindungen, die in der allgemeinen Formel (I) nicht gesondert angegeben sind. Je nach der spezifischen Raumform werden die möglichen Stereoisomeren als Enantiomere, Diastereomere, Z- und E-Isomere bezeichnet und können nach üblichen Methoden aus Gemischen der Stereoisomeren erhalten oder auch durch stereoselektive Reaktionen in Kombination mit dem Einsatz von stereochemisch reinen Ausgangsstoffen hergestellt werden.

Die Verbindungen der Formel (I) können Salze bilden, bei denen der Wasserstoff der -SO₂-NH-Gruppe durch ein für die Landwirtschaft geeignetes Kation ersetzt wird. Diese Salze sind beispielsweise Metallsalze, vorzugsweise Alkali- oder Erdalkalisalze, insbesondere Natrium und Kaliumsalze, oder auch Ammoniumsalze oder Salze mit organischen Aminen. Ebenso kann Salzbildung durch Anlagerung einer Säure an basische Gruppen, wie z.B. Amino und Alkylamino, erfolgen. Geeignete Säuren hierfür sind starke anorganische und organische Säuren, beispielsweise HCI, HBr, H₂SO₄ oder HNO₃.

Die Verbindungen der Formel (I) sind nach literaturbekannten Verfahren zugänglich (US-A-4,369,058, US-A-4,453,971, US-A-4,225,237).

Die Salze der erfindungsgemäßen Verbindungen (I) mit Basen stellen neue Verbindungen dar. Sie sind ebenfalls Gegenstand dieser Erfindung.

Die Salze der Verbindungen der Formel (I) werden vorzugsweise in unter den Herstellbedingungen inerten anorganischen oder organischen Lösungsmitteln, z.B. Wasser, Alkoholen wie Methanol, Ketonen wie Aceton, gegebenenfalls halogenierten aliphatischen oder aromatischen Kohlenwasserstoffen wie Heptan, Dichlormethan, Toluol oder Chlorbenzol oder Ethern wie Tetrahydrofuran, bei Temperaturen zwischen dem Festpunkt bis zum Siedepunkt des jeweiligen Lösungsmittels, vorzugsweise bei Temperaturen von 0 bis 100°C, durch Umsetzung der Sulfonylharnstoffe mit Säuren oder Basen hergestellt. Geeignete Basen zur Herstellung der erfindungsgemäßen Salze sind beispielsweise Alkalicarbonate, wie Kaliumcarbonat, Alkali- und Erdalkalihydroxide, wie NaOH, KOH und Ca(OH)₂, Ammoniak oder eine geeignete Aminbase, wie Triethylamin oder Ethanolamin.

Geeignete Säuren zur Salzbildung wurden bereits oben erwähnt.

Die erfindungsgemäßen Verbindungen der Formel (I) oder deren Salze weisen eine ausgezeichnete herbizide Wirksamkeit gegen ein breites Spektrum wirtschaftlich wichtiger mono- und dikotyler Schadpflanzen auf. Auch schwer bekämpfbare perennierende Unkräuter, die aus Rhizomen, Wurzelstöcken oder anderen Dauerorganen austreiben, werden durch die Wirkstoffe gut erfaßt. Dabei ist es gleichgültig, ob die Substanzen im Vorsaat-, Vorauflauf- oder Nachauflaufverfahren ausgebracht werden. Im einzelnen seien beispielsweise einige Vertreter der mono- und dikotylen Unkrautflora genannt, die durch die erfindungsgemäßen Verbindungen kontrolliert werden können, ohne daß durch die Nennung eine Beschränkung auf bestimmte Arten erfolgen soll.

Auf der Seite der monokotylen Unkrautarten werden z.B. Avena, Lolium, Alopecurus, Phalaris, Echinochloa, Digitaria, Setaria sowie Cyperusarten aus der annuellen Gruppe und auf seiten der perennierenden Spezies Agropyron, Cynodon, Imperata sowie Sorghum und auch ausdauernde Cyperusarten gut erfaßt.
Bei dikotylen Unkrautarten erstreckt sich das Wirkungsspektrum auf Arten wie z.B. Galium, Viola, Veronica, Lamium, Stellaria, Amaranthus, Sinapis, Ipomoea, Matricaria, Abutilon und Sida auf der annuellen Seite sowie Convolvulus, Cirsium, Rumex und Artemisia bei den perennierenden Unkräutern.

Speziell unter den spezifischen Kulturbedingungen im Reis vorkommende Unkräuter wie z.B. Sagittaria, Alisma, Eleocharis, Scirpus und Cyperus werden von den erfindungsgemäßen Wirkstoffen hervorragend bekämpft.

Werden die erfindungsgemäßen Verbindungen vor dem Keimen auf die Erdoberfläche bzw. in das Anstauwasser beim Reisanbau appliziert, so wird entweder das Auflaufen der Unkrautkeimlinge vollständig verhindert oder die Unkräuter wachsen bis zum Keimblattstadium heran, stellen jedoch dann ihr Wachstum ein und sterben schließlich nach Ablauf von drei bis vier Wochen vollkommen ab.

Bei Applikation der Wirkstoffe auf die grünen Pflanzenteile im Nachauflaufverfahren tritt ebenfalls sehr rasch nach der Behandlung ein drastischer Wachstumsstop ein und die Unkrautpflanzen bleiben in dem zum Applikationszeitpunkt vorhandenen Wachstumsstadium stehen oder sterben nach einer gewissen Zeit ganz ab, so daß auf diese Weise eine für die Kulturpflanzen schädliche Unkrautkonkurrenz sehr früh und nachhaltig beseitigt wird.

Obgleich die Verbindungen der Formel (I) oder deren Salze eine ausgezeichnete herbizide Aktivität gegenüber mono- und dikotylen Unkräutern aufweisen, werden Reiskulturen aus gesätem oder verpflanzten Reis nur unwesentlich oder gar nicht geschädigt. Die vorliegenden Verbindungen eignen sich aus diesen Gründen sehr gut zur selektiven Bekämpfung von unerwünschtem Pflanzenwuchs in landwirtschaftlichen Nutzpflanzungen.

Darüber hinaus weisen einige der Verbindungen wachstumsregulatorische Eigenschaften bei den Reispflanzen auf. Sie greifen regulierend in den pflanzeneigenen Stoffwechsel ein und können damit zur gezielten Beeinflussung von Pflanzeninhaltsstoffen und zur Ernteerleichterung, z.B. durch Auslösen von Desikkation und Wuchsstauchung oder zur Steuerung oder Hemmung von unerwünschten vegetativen Wachstum, eingesetzt werden.

Die Verbindungen der Formel (I) oder deren Salze können in Form von Spritzpulvern, emulgierbaren Konzentraten, versprühbaren Lösungen, Stäubemitteln oder Granulaten in den üblichen Zubereitungen angewendet werden. Gegenstand der Erfindung sind deshalb auch herbizide und pflanzenwachstumsregulierende Mittel, die die neuen Salze der Verbindungen der Formel (I) enthalten.

Die Verbindungen der Formel (I) oder deren Salze können auf verschiedene Art formuliert werden, je nachdem welche biologischen und/oder chemischphysikalischen Parameter vorgegeben sind. Als Formulierungsmöglichkeiten kommen beispielsweise in Frage: Spritzpulver (WP), wasserlösliche Pulver (SP), wasserlösliche Konzentrate, emulgierbare Konzentrate (EC), Emulsionen (EW), wie Öl-in-Wasser- und Wasser-in-Öl-Emulsionen, versprühbare Lösungen, Suspensionskonzentrate (SC), Dispersionen auf Öl- oder Wasserbasis, ölmischbare Lösungen, Kapselsuspensionen (CS), Stäubemittel (DP), Beizmittel, Granulate für die Streu- und Bodenapplikation, Granulate (GR) in Form von Mikro-, Sprüh-, Aufzugs- und Adsorptionsgranulaten, wasserdispergierbare Granulate (WG), wasserlösliche Granulate (SG), ULV-Formulierungen, Mikrokapseln und Wachse.

Diese einzelnen Formulierungstypen sind im Prinzip bekannt und werden beispielsweise beschrieben in: Winnacker-Küchler, "Chemische Technologie", Band 7, C. Hauser Verlag München, 4. Aufl. 1986, Wade van Valkenburg, "Pesticide Formulations", Marcel Dekker, N.Y., 1973; K. Martens, "Spray Drying" Handbook, 3rd Ed. 1979, G. Goodwin Ltd. London.

Die notwendigen Formulierungshilfsmittel wie Inertmaterialien, Tenside, Lösungsmittel und weitere Zusatzstoffe sind ebenfalls bekannt und werden beispielsweise beschrieben in: Watkins, "Handbook of Insecticide Dust Diluents and Carriers", 2nd Ed., Darland Books, Caldwell N.J., H.v. Olphen, "Introduction to Clay Colloid Chemistry"; 2nd Ed., J. Wiley & Sons, N.Y.; C. Marsden, "Solvents Guide"; 2nd Ed., Interscience, N.Y. 1963; McCutcheon's "Detergents and Emulsifiers Annual", MC Publ. Corp., Ridgewood N.J.; Sisley and Wood, "Encyclopedia of Surface Active Agents", Chem. Publ. Co. Inc., N.Y. 1964; Schönfeldt, "Grenzflächenaktive Äthylenoxidaddukte", Wiss. Verlagsgesell., Stuttgart 1976; Winnacker-Küchler, "Chemische Technologie", Band 7, C. Hauser Verlag München, 4. Aufl. 1986.

Auf der Basis dieser Formulierungen lassen sich auch Kombinationen mit anderen pestizid wirksamen Stoffen, wie z.B. Insektiziden, Akariziden, anderen Herbiziden, Fungiziden, Safenern, Wachstumsregulatoren und/oder Düngemitteln herstellen, z.B. in Form einer Fertigformulierung oder als Tankmix.

Spritzpulver sind in Wasser gleichmäßig dispergierbare Präparate, die neben dem Wirkstoff außer einem Verdünnungs- oder Inertstoff noch Tenside ionischer und/oder nichtionischer Art (Netzmittel, Dispergiermittel), z.B. polyoxyethylierte Alkylphenole, polyoxethylierte Fettalkohole, polyoxethylierte Fettamine, Fettalkoholpolyglykolethersulfate, Alkansulfonate, Alkylbenzolsulfonate, ligninsulfonsaures Natrium, 2,2'-dinaphthylmethan-6,6'-disulfonsaures Natrium, dibutylnaphthalin-sulfonsaures Natrium oder auch oleoylmethyltaurinsaures Natrium enthalten. Zur Herstellung der Spritzpulver werden die herbiziden Wirkstoffe beispielsweise in üblichen Apparaturen wie Hammermühlen, Gebläsemühlen und Luftstrahlmühlen feingemahlen und gleichzeitig oder anschließend mit den Formulierungshilfsmitteln vermischt.

Emulgierbare Konzentrate werden durch Auflösen des Wirkstoffes in einem organischen Lösungsmittel z.B. Butanol, Cyclohexanon, Dimethylformamid, Xylol oder auch höhersiedenden Aromaten oder Kohlenwasserstoffen oder Mischungen der organischen Lösungsmittel unter Zusatz von einem oder mehreren Tensiden ionischer und/oder nichtionischer Art (Emulgatoren) hergestellt. Als Emulgatoren können beispielsweise verwendet werden: Alkylarylsulfonsaure Calcium-Salze wie
Ca-dodecylbenzolsulfonat oder nichtionische Emulgatoren wie Fettsäurepolyglykolester, Alkylarylpolyglykolether, Fettalkoholpolyglykolether, Propylenoxid-Ethylenoxid-Kondensationsprodukte, Alkylpolyether, Sorbitanester wie z. B. Sorbitanfettsäureester oder Polyoxethylensorbitanester wie z. B. Polyoxyethylensorbitanfettsäureester.

Stäubemittel erhält man durch Vermahlen des Wirkstoffes mit fein verteilten festen Stoffen, z.B. Talkum, natürlichen Tonen, wie Kaolin, Bentonit und Pyrophyllit, oder Diatomeenerde.

Suspensionskonzentrate können auf Wasser- oder Ölbasis sein. Sie können beispielsweise durch Naß-Vermahlung mittels handelsüblicher Perlmühlen und gegebenenfalls Zusatz von Tensiden, wie sie z. B. oben bei den anderen Formulierungstypen bereits aufgeführt sind, hergestellt werden.

Emulsionen, z. B. Öl-in-Wasser-Emulsionen (EW), lassen sich beispielsweise mittels Rührern, Kolloidmühlen und/oder statischen Mischern unter Verwendung von wäßrigen organischen Lösungsmitteln und gegebenenfalls Tensiden, wie sie z. B. oben bei den anderen Formulierungstypen bereits aufgeführt sind, herstellen.

Granulate können entweder durch Verdüsen des Wirkstoffes auf adsorptionsfähiges, granuliertes Inertmaterial hergestellt werden oder durch Aufbringen von Wirkstoffkonzentraten mittels Klebemitteln, z.B. Polyvinylalkohol, polyacrylsaurem Natrium oder auch Mineralölen, auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise - gewünschtenfalls in Mischung mit Düngemitteln - granuliert werden.

Wasserdispergierbare Granulate werden in der Regel nach den üblichen Verfahren wie Sprühtrocknung, Wirbelbett-Granulierung, Teller-Granulierung, Mischung mit Hochgeschwindigkeitsmischern und Extrusion ohne festes Inertmaterial hergestellt.

Die agrochemischen Zubereitungen enthalten in der Regel 0,1 bis 99 Gew.-%, insbesondere 0,1 bis 95 Gew.-%, Wirkstoff der Formel (I) oder deren Salze.

In Spritzpulvern beträgt die Wirkstoffkonzentration z.B. etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 1 bis 90, vorzugsweise 5 bis 80 Gew.-% betragen. Staubförmige Formulierungen enthalten 1 bis 30, vorzugsweise meistens 5 bis 20 Gew.-% an Wirkstoff. versprühbare Lösungen etwa 0,05 bis 80, vorzugsweise 2 bis 50 Gew.-% Wirkstoff. Bei wasserdispergierbaren Granulaten hängt der Wirkstoffgehalt zum Teil davon ab, ob die wirksame Verbindung flüssig oder fest vorliegt und welche Granulierhilfsmittel, Füllstoffe usw. verwendet werden. Bei den in Wasser dispergierbaren Granulaten liegt der Gehalt an Wirkstoff beispielsweise zwischen 1 und 95 Gew.-%, vorzugsweise zwischen 10 und 80 Gew.-%.

Daneben enthalten die genannten Wirkstofformulierungen gegebenenfalls die jeweils üblichen Haft-, Netz-, Dispergier-, Emulgier-, Penetrations-, Konservierungs-, Frostschutz- und Lösungsmittel, Füll-, Träger- und Farbstoffe, Entschäumer, Verdunstungshemmer und den pH-Wert und die Viskosität beeinflussende Mittel.

Als Kombinationspartner für die erfindungsgemäßen Wirkstoffe in Mischungsformulierungen oder im Tank-Mix sind beispielsweise bekannte Wirkstoffe einsetzbar, wie sie z.B. in Weed Research 26, 441-445 (1986), oder "The Pesticide Manual", 9th edition, The British Crop Protection Council, 1990/91, Bracknell, England, und dort zitierter Literatur für die Anwendung in Reiskulturen beschrieben sind.

Zur Anwendung werden die in handelsüblicher Form vorliegenden Formulierungen gegebenenfalls in üblicher Weise verdünnt, z.B. bei Spritzpulvern, emulgierbaren Konzentraten, Dispersionen und wasserdispergierbaren Granulaten mittels Wasser, und anschließend auf die Pflanzen, Pflanzenteile oder den landwirtschaftlich oder industriell genützten Boden oder die Anbaufläche, auf dem die Pflanzen stehen oder in dem sie heranwachsen oder als Saat vorliegen, appliziert. Dies schließt auch spezielle Applikationsvarianten ein, wie sie im Reisanbau üblich sind, z.B. die Gießapplikation, bei der die Applikation der herbiziden Mittel in das Anstauwasser der bewässerten Anbaufläche erfolgt. Staubförmige Zubereitungen, Boden- bzw. Streugranulate sowie versprühbare Lösungen werden vor der Anwendung üblicherweise nicht mehr mit weiteren inerten Stoffen verdünnt.

Mit den äußeren Bedingungen wie Temperatur, Feuchtigkeit, der Art des verwendeten Herbizids, u.a. variiert die erforderliche Aufwandmenge der Verbindungen der Formel (I) oder deren Salze. Sie kann innerhalb weiter Grenzen schwanken, z.B. zwischen 0,0001 und 0,5 kg/ha oder mehr Aktivsubstanz, vorzugsweise liegt sie jedoch zwischen 0,005 und 0,12 kg/ha.

### A. Chemische Beispiele

### Beispiel A1: N-[(4,6-Dimethoxypyrimidin-2-yl)-aminocarbonyl]-2-methoxycarbonylamino-benzolsulfonamid (Tabelle 1, Bsp.-Nr. 6)

3,0 g 2-(Methoxycarbonylamino)-benzolsulfonamid und 7,1 g 4,6-Dimethoxy-2-(phenoxycarbonylamino)-pyrimidin werden in 30 ml Acetonitril vorgelegt, auf 0 bis 5°C abgekühlt und mit 4,9 ml 1,8-Diazabicyclo[5.4.O]undec-7-en (DBU) versetzt. Nach beendeter Reaktion wird das Gemisch eingeengt und in Wasser aufgenommen. Nach dem Waschen mit Diethylether wird die wäßrige Phase mit konzentrierter Salzsäure angesäuert (pH = 1 - 2). Der abgeschiedene Feststoff wird durch Ausrühren mit Methanol und Diisopropylether gereinigt. Man erhält so 4,5 g des gewünschten Produkts (siehe Titel zu A1); Schmelzpunkt: 195 bis 196°C (Z).

### Beispiel A2: N-[(4,6-Dimethoxypyrimidin-2-yl)-aminocarbonyl]-2-methoxycarbonylamino-benzolsulfonamid-natriumsalz (Tabelle 1, Bsp.-Nr. 9)

1,0 g N-[(4,6-Dimethoxypyrimidin-2-yl)-aminocarbonyl]-2-(methoxycarbonylamino)-benzolsulfonamid (Bsp. A1) werden in 20 ml Methylenchlorid vorgelegt und mit 0,1 g Natriumhydroxid versetzt. Nach beendeter Reaktion wird das Gemisch unter reduziertem Druck eingeengt. Man erhält so 1,1 g des gewünschten Salzes (siehe Titel zu A2); Schmelzpunkt: 170-173°C (Z).

Die in der nachfolgenden Tabelle 1 zusammen mit den Produkten der obigen Beispiele A1 und A2 aufgeführten Verbindungen werden nach bzw. in analoger Weise zu den Beispielen A1 und A2, gegebenenfalls unter Anwendung der in der allgemeinen Beschreibung erwähnten Verfahren, erhalten. In der Tabelle 1 bedeuten:
- Bsp.-Nr. =: Beispiel Nummer/Verbindung Nummer
- Smp. °C =: Schmelzpunkt in Grad Celsius
- Me =: Methyl
- Et =: Ethyl
- Pr =: n-Propyl
- n-Pr =: n-Propyl
- i-Pr =: Isopropyl
- c-Pr =: Cyclopropyl
In Formel (Ia) steht "M" im Falle "H" für kovalent gebundenes H und ansonsten für das Äquivalent eines Kations mit der in der Tabelle 1 angegebenen Formel, wobei die positive Ladung nicht angegeben ist.

Einige der Verbindungen konnten nicht kristallin oder lösungsmittelfrei erhalten werden und wurden NMR-spektroskopisch identifiziert.

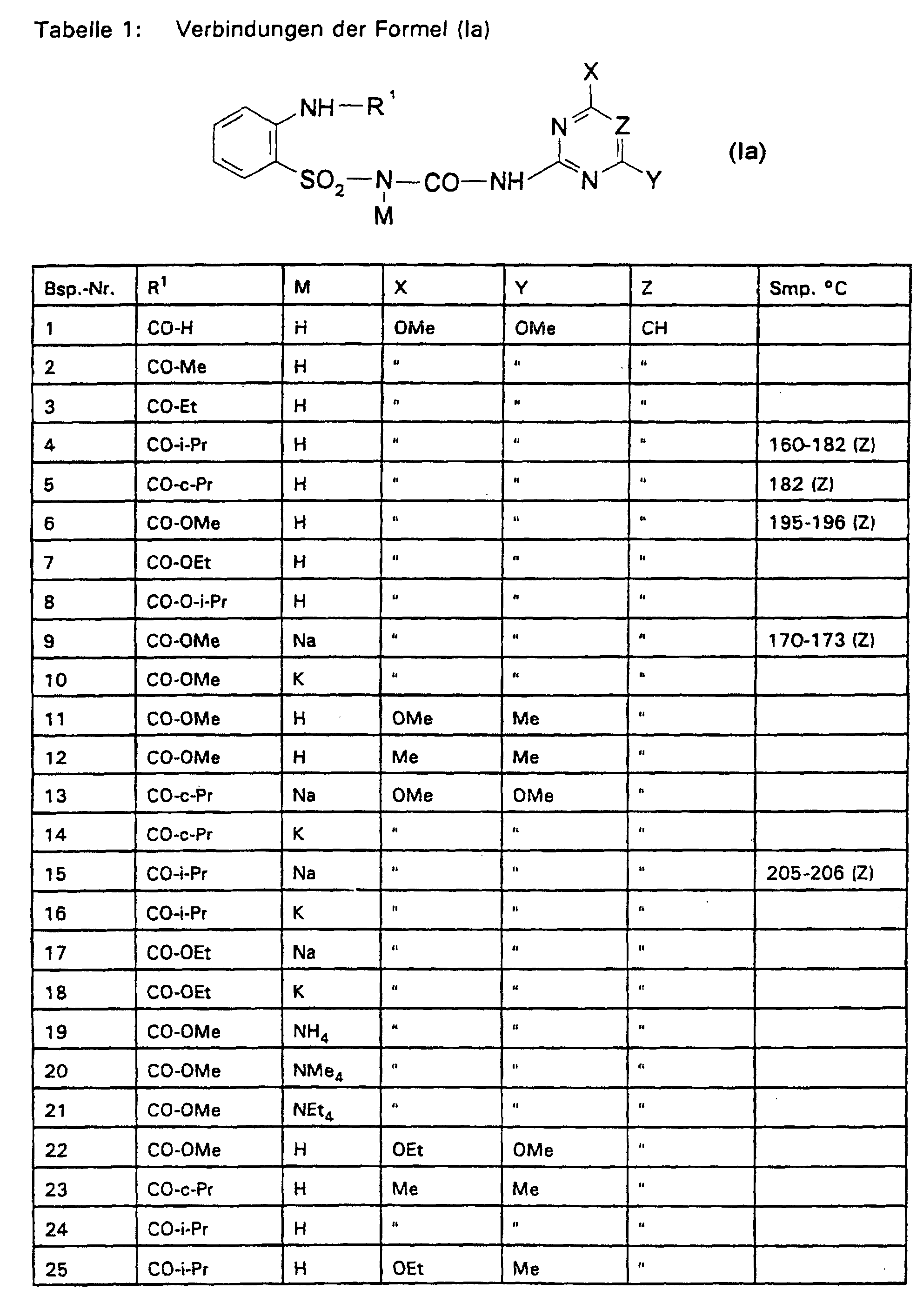

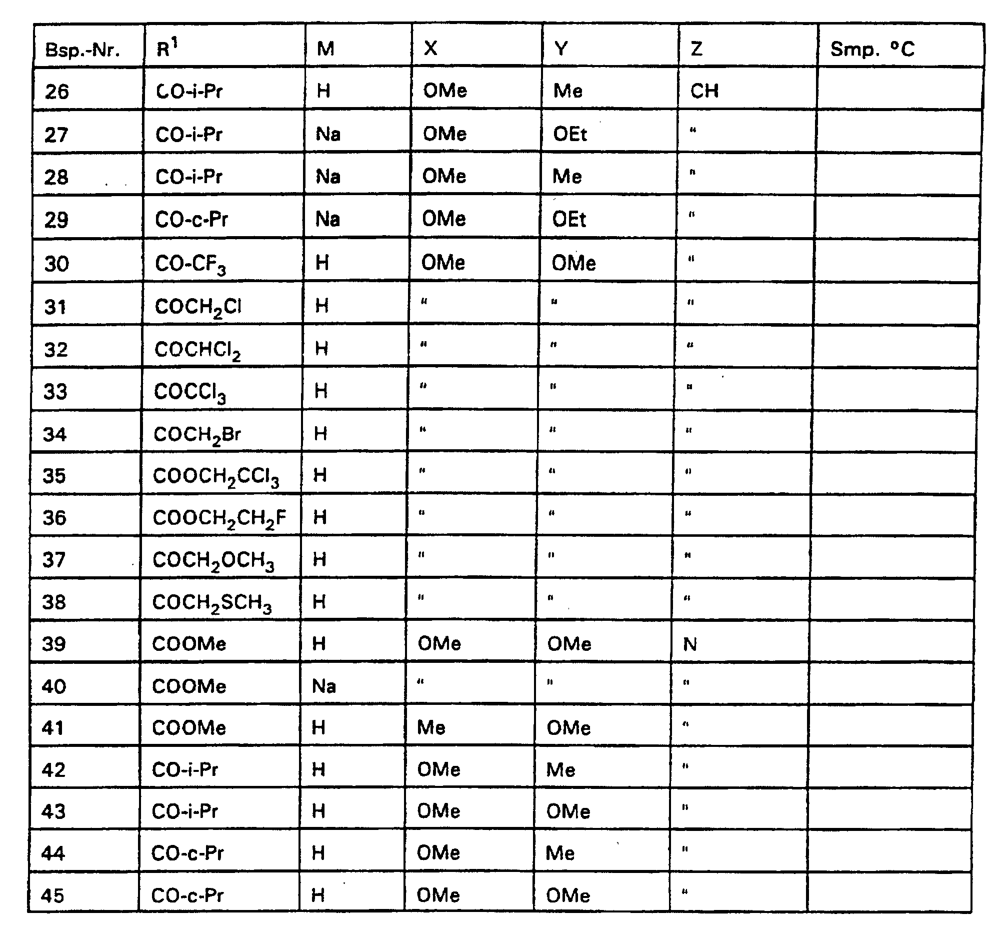

### B. Formulierungsbeispiele

a) Ein Stäubemittel wird erhalten, indem man 10 Gew.-Teile einer Verbindung der Formel (I) und 90 Gew.-Teile Talkum als Inertstoff mischt und in einer Schlagmühle zerkleinert.
b) Ein in Wasser leicht dispergierbares, benetzbares Pulver wird erhalten, indem man 25 Gewichtsteile einer Verbindung der Formel (I), 64 Gewichtsteile kaolinhaltigen Quarz als Inertstoff, 10 Gewichtsteile ligninsulfonsaures Kalium und 1 Gewichtsteil oleoylmethyltaurinsaures Natrium als Netz- und Dispergiermittel mischt und in einer Stiftmühle mahlt.
c) Ein in Wasser leicht dispergierbares Dispersionskonzentrat wird erhalten, indem man 20 Gewichtsteile einer Verbindung der Formel (I) mit 6 Gewichtsteilen Alkylphenolpolyglykolether (®Triton X 207), 3 Gewichtsteilen Isotridecanolpolyglykolether (8 EO) und 71 Gewichtsteilen paraffinischem Mineralöl (Siedebereich z.B. ca. 255 bis über 277°C) mischt und in einer Reibkugelmühle auf eine Feinheit von unter 5 Mikron vermahlt.
d) Ein emulgierbares Konzentrat wird erhalten aus 15 Gewichtsteilen einer Verbindung der Formel (I), 75 Gewichtsteilen Cyclohexanon als Lösungsmittel und 10 Gewichtsteilen oxethyliertes Nonylphenol als Emulgator.
e) Ein in Wasser dispergierbares Granulat wird erhalten indem man
   75 Gewichtsteile einer Verbindung der Formel (I),
   10 Gewichtsteile ligninsulfonsaures Calcium,
   5 Gewichtsteile Natriumlaurylsulfat,
   3 Gewichtsteile Polyvinylalkohol und
   7 Gewichtsteile Kaolin
   mischt, auf einer Stiftmühle mahlt und das Pulver in einem Wirbelbett durch Aufsprühen von Wasser als Granulierflüssigkeit granuliert.
f) Ein in Wasser dispergierbares Granulat wird auch erhalten, indem man
   25 Gewichtsteile einer Verbindung der Formel (I),
   5 Gewichtsteile 2,2'-dinaphthylmethan-6,6'-disulfonsaures Natrium
   2 Gewichtsteile oleoylmethyltaurinsaures Natrium,
   1 Gewichtsteil Polyvinylalkohol,
   17 Gewichtsteile Calciumcarbonat und
   50 Gewichtsteile Wasser
   auf einer Kolloidmühle homogenisiert und vorzerkleinert, anschließend auf einer Perlmühle mahlt und die so erhaltene Suspension in einem Sprühturm mittels einer Einstoffdüse zerstäubt und trocknet.

### C. Biologische Beispiele

### Wirkung auf Schadpflanzen in Reis

Verpflanzter und gesäter Reis sowie typische Reisunkräuter werden im Gewächshaus bis zum Dreiblattstadium (Echinochloa 1,5-Blatt) unter Paddyreis-Bedingungen (Anstauhöhe des Wassers: 2 - 3 cm) in geschlossenen Plastiktöpfen angezogen. Danach erfolgt die Behandlung mit den Verbindungen der Formel (I) bzw. deren Salzen. Hierzu werden die formulierten Wirkstoffe in Wasser suspendiert, gelöst bzw. emulgiert und mittels Gießapplikation in das Anstauwasser der Test-pflanzen in unterschiedlichen Dosierungen ausgebracht. Nach der so durchgeführten Behandlung werden die Versuchspflanzen im Gewächshaus unter optimalen Wachstumsbedingungen aufgestellt und während der gesamten Versuchszeit so gehalten.

Etwa drei Wochen nach der Applikation erfolgt die Auswertung mittels optischer Bonitur der Pflanzenschäden im Vergleich zu unbehandelten Kontrollen, wobei die Verbindungen der Formel (I) und deren Salze sehr gute herbizide Wirkung gegen Schadpflanzen zeigen, die typisch für Reiskulturen sind, und die Reispflanzen vor allem bei niedrigen Aufwandmengen nur unwesentlich oder gar nicht schädigen. Einige strukturell sehr nahe Vergleichsverbindungen zeigen dagegen überraschenderweise keine oder wesentlich schlechtere Selektivität. In Tabelle 2 sind eine Reihe der Testergebnisse zusammengefaßt.

**Tabelle 2:**

| Herbizide Wirkung und Selektivität in Reis | | | | | | | |
|---|---|---|---|---|---|---|---|
| Bsp. | Dosis g a.i./ha | Herbizide Wirkung in % bei | | | | | |
| | | ECHCG | SAGPY | ELOAC | CYPSE | ORSA/V | ORSA/S |
| 4 | 120 | 40 | 90 | 85 | 85 | 25 | 0 |
| | 60 | 25 | 90 | 80 | 70 | 20 | 0 |
| | 30 | 30 | 90 | 80 | 50 | 15 | 0 |
| | 15 | 15 | 90 | 80 | 20 | 0 | 0 |
| 5 | 120 | 75 | 95 | 90 | 85 | 30 | 10 |
| | 60 | 70 | 90 | 90 | 90 | 15 | 0 |
| | 30 | 55 | 80 | 90 | 90 | 0 | 0 |
| | 15 | 35 | 70 | 80 | 70 | 0 | 0 |
| 6 | 120 | 90 | 90 | 90 | 95 | 40 | 40 |
| | 60 | 80 | 90 | 90 | 95 | 25 | 30 |
| | 30 | 55 | 90 | 90 | 95 | 25 | 20 |
| 9 | 120 | 97 | 80 | 85 | 95 | 35 | 35 |
| | 60 | 93 | 80 | 80 | 95 | 15 | 20 |
| | 30 | 88 | 85 | 80 | 95 | 15 | 0 |
| | 15 | 70 | 80 | 80 | 95 | 0 | 0 |
| 16 | 120 | 65 | 85 | 85 | 80 | 50 | 15 |
| | 60 | 65 | 80 | 80 | 70 | 43 | 0 |
| | 30 | 40 | 85 | 80 | 75 | 7 | 0 |
| | 15 | 35 | 80 | 80 | 30 | 5 | 0 |
| V | 120 | 96 | 80 | 88 | 90 | 80 | 80 |
| | 60 | 98 | 70 | 85 | 85 | 80 | 75 |
| | 30 | 95 | 70 | 80 | 85 | 70 | 65 |
| | 15 | 98 | 70 | 70 | 80 | 65 | 60 |

### Abkürzungen zu Tabelle 2: Siehe folgende Seite

Abkürzungen zu Tabelle 2:
- g a.i./ha =: Gramm Aktivsubstanz ("active ingredient") pro Hektar, bezogen auf 100% reinen Wirkstoff
- ECHCG =: Echinocloa crus-galli
- SAGPY =: Sagittaria pygmaea
- ELEOAC =: Eleocharis acicularis
- CYPSE =: Cyperus serotinus
- ORYSA/V =: Oryza sativa (verpflanzt), Reis
- ORYSA/S =: Oryza sativa (gesät), Reis
- Bsp. (Nr.) =: Beispiel (Nummer)/Verbindung (Nummer), wobei im Falle einer einfachen Zahl das Beispiel aus Tabelle 1 mit derselben Nummer bezeichnet wird
- Bsp. V =: Vergleichsverbindung N-[(4,6-Dimethoxypyrimidin-2-yl)-aminocarbonyl]-2-aminobenzolsulfonamid

## Patentansprüche

1. Verfahren zur Bekämpfung von Schadpflanzen in Reiskulturen, **dadurch gekennzeichnet, daß** man eine oder mehrere Verbindungen der Formel (I) oder deren Salze, 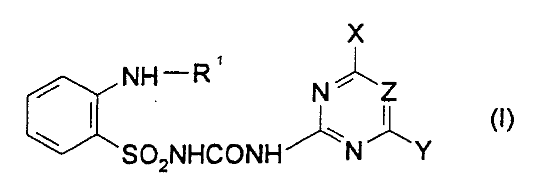 worin
R¹ einen Acylrest aus der Gruppe CO-R², CS-R², CO-OR³, CS-OR³, CO-SR⁴, CS-SR⁴, CO-NR⁵R⁶ und CS-NR⁵R⁶,
R² H, (C₁-C₆)Alkyl, (C₃-C₆)Cycloalkyl, (C₃-C₆)Cycloalkyl-(C₁-C₄)alkyt, (C₂-C₆)Alkenyl oder (C₂-C₆)Alkinyl, wobei jeder der 5 letztgenannten Reste unsubstituiert oder durch einen oder mehrere Reste aus der Gruppe Halogen, (C₁-C₄)Alkoxy, (C₁-C₄)Haloalkoxy, (C₁-C₄)Alkylthio, (C₁-C₄)Haloalkylthio, CN, NH₂, Mono- und Di(C₁-C₄)alkylamino und im Falle cyclischer Reste auch (C₁-C₄)Alkyl und (C₁-C₄)Haloalkyl substituiert ist,
R³ einen Rest aus der Gruppe der für R² möglichen Reste, außer H,
R⁴ einen Rest aus der Gruppe der für R³ möglichen Reste,
R⁵,R⁶ unabhängig voneinander jeweils einen Rest aus der Gruppe der für R² möglichen Reste oder die Gruppe
NR⁵R⁶ gemeinsam einen am N-Atom gebundenen substituierten oder unsubstituierten 4-, 5- oder 6-gliedrigen gesättigten oder ungesättigten heterocyclischen Rest, der neben dem N-Atom kein weiteres Heteroringatom oder weitere Heteroringatome enthält und der unsubstituiert oder durch einen oder mehrere Reste aus der Gruppe (C₁-C₄)Alkyl, (C₁-C₄)Haloalkyl, Halogen, (C₁-C₄)Alkoxy und (C₁-C₄)Haloalkoxy substituiert ist,
X,Y unabhängig voneinander Halogen, (C₁-C₆)Alkyl, (C₁-C₆)Alkoxy, (C₁-C₆)Alkylthio, wobei jeder der drei letztgenannten Reste unsubstituiert oder durch einen oder mehrere Reste aus der Gruppe Halogen, (C₁-C₄)Alkoxy und (C₁-C₄)Alkylthio substituiert ist, oder (C₃-C₆)Cycloalkyl, (C₃-C₆)Alkenyl, (C₃-C₆)Alkinyl, (C₃-C₆)Alkenyloxy oder (C₃-C₆)Alkinyloxy und
Z CH oder N
bedeuten,
auf die Anbaufläche, welche Schad- und Reispflanzen oder deren Samen enthält, in einer wirksamen Menge von 0,0001 bis 0,5 kg a.i./ha appliziert.

2. Verfahren nach Anspruch 1, **dadurch gekennzeichnet, daß**
R¹ CO-R², CS-R², CO-OR³, CS-OR³, CO-SR⁴, CS-SR⁴, CO-NR⁵R⁶ und CS-NR⁵R⁶,
R² H, (C₁-C₆)Alkyl, (C₃-C₆)Cycloalkyl, (C₃-C₆)Cycloalkyl-(C₁-C₂)alkyl, (C₂-C₄)Alkenyl oder (C₂-C₄)Alkinyl, wobei jeder der 5 letztgenannten Reste unsubstituiert oder durch einen oder mehrere Reste aus der Gruppe Halogen, (C₁-C₃)Alkoxy, (C₁-C₃)Haloalkoxy, (C₁-C₃)Alkylthio, (C₁-C₃)Haloalkylthio, CN, NH₂, Mono- und Di(C₁-C₃)alkylamino und im Falle cyclischer Reste auch (C₁-C₄)Alkyl und (C₁-C₄)Haloalkyl substituiert ist,
R³ einen Rest aus der Gruppe der für R² möglichen Reste, außer H,
R⁴ einen Rest aus der Gruppe der für R³ möglichen Reste,
R⁵,R⁶ unabhängig voneinander jeweils einen Rest aus der Gruppe der für R² möglichen Reste oder die Gruppe
NR⁵R⁶ gemeinsam einen am N-Atom gebundenen substituierten oder unsubstituierten 4-, 5- oder 6-gliedrigen gesättigten heterocyclischen Rest der Formel (A-1), (A-2), (A-3) oder (A-4) 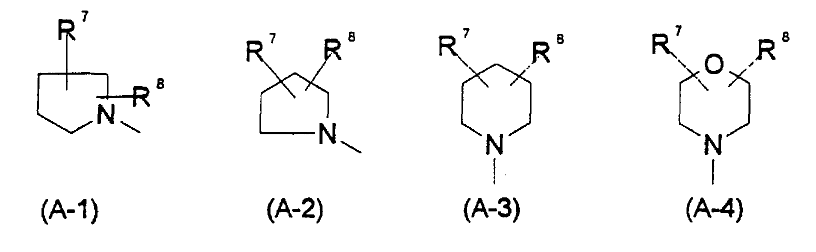
R⁷,R⁸ unabhängig voneinander H, Halogen, (C₁-C₃)Alkyl, (C₁-C₃)Haloalkyl, (C₁-C₃)Alkoxy oder (C₁-C₃)Haloalkoxy,
X,Y unabhängig voneinander Halogen, (C₁-C₄)Alkyl, (C₁-C₄)Alkoxy, (C₁-C₄)Alkylthio, wobei jeder der drei letztgenannten Reste unsubstituiert oder durch einen oder mehrere Reste aus der Gruppe Halogen, (C₁-C₄)Alkoxy und (C₁-C₄)Alkylthio substituiert ist, oder (C₃-C₆)Cycloalkyl, (C₃-C₄)Alkenyl, (C₃-C₄)Alkinyl, (C₃-C₄)Alkenoxy oder (C₃-C₄)Alkinoxy und
Z CH oder N bedeuten.

3. Verfahren nach Anspruch 1 oder 2, **dadurch gekennzeichnet, daß**
R² H, (C₁-C₄)Alkyl, (C₁-C₄)Haloalkyl, (C₁-C₃)Alkoxy-(C₁-C₄)alkyl, (C₁-C₃)Alkylthio-(C₁-C₄)alkyl, (C₃-C₆)Cycloalkyl oder (C₃-C₆)Cycloalkylmethyl,
R³ (C₁-C₄)Alkyl, (C₁-C₄)Haloalkyl, (C₁-C₃)Alkoxy-(C₁-C₄)alkyl, (C₁-C₃)Alkylthio-(C₁-C₄)alkyl, (C₃-C₆)Cycloalkyl, (C₃-C₆)Cycloalkylmethyl, (C₃-C₄)Alkenyl oder (C₃-C₄)Alkinyl,
R⁴ einen Rest aus der Gruppe der für R³ möglichen Reste,
R⁵,R⁶ unabhängig voneinander H oder (C₁-C₄)Alkyl, einer der Reste X und Y (C₁-C₄)Alkyl, (C₁-C₄)Alkoxy, (C₁-C₄)Haloalkyl oder (C₁-C₄)Haloalkoxy,
und der andere der Reste X und Y Halogen, (C₁-C₄)Alkyl, (C₁-C₄)Alkoxy, (C₁-C₄)Alkylthio, wobei die drei letztgenannten Reste unsubstituiert oder durch einen oder mehrere Reste aus der Gruppe Halogen, (C₁-C₄)Alkoxy und (C₁-C₄)Alkylthio substituiert sind, oder (C₃-C₆)Cycloalkyl, (C₂-C₄)Alkenyl, (C₂-C₄)Alkinyl, (C₃-C₄)Alkenoxy oder (C₃-C₄)Alkinoxy und
Z CH oder N
bedeuten.

4. Verfahren nach einem der Ansprüche 1 bis 3, **dadurch gekennzeichnet, daß**
R¹ COR² oder CO-OR³,
R² H, (C₁-C₄)Alkyl oder (C₃-C₄)Cycloalkyl.
R³ (C₁-C₄)Alkyl oder (C₃-C₆)Cycloalkyl,
einer der Reste X und Y Methoxy, Ethoxy oder Methyl und
der andere der Reste X und Y Methoxy, Ethoxy, Methyl, Chlor, Fluor oder CF₃ und
Z CH oder N bedeuten.

5. Verfahren nach Anspruch 4, **dadurch gekennzeichnet, daß**
R² Ethyl, Isopropyl oder Cyclopropyl,
R³ Methyl oder Ethyl,
X, Y jeweils Methoxy und
Z CH bedeuten.

6. Verfahren nach einem der Ansprüche 1 bis 5, **dadurch gekennzeichnet, daß** man die Verbindungen (I) oder deren Salze in einer Aufwandmenge von 0,001 bis 0,2 kg a.i./ha appliziert.

7. Salze der Formel (Ia) 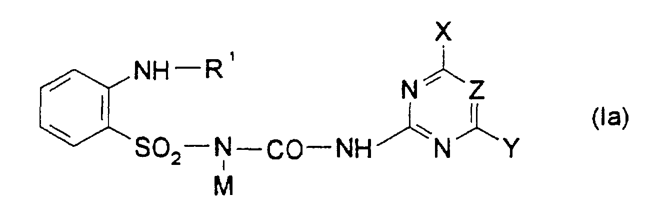 worin
M das Äquivalent eines Kations ist und
R¹, R², R³, R⁴, X und Y wie in Formel (I) nach einem der Ansprüche 1 bis 5 definiert sind.

8. Verfahren zur Herstellung der Salze nach Anspruch 7, **dadurch gekennzeichnet, daß** man die Verbindungen der Formel (Ia), worin M = H bedeutet, mit Basen umsetzt.

9. Verwendung von Verbindungen der Formeln (I) oder (Ia) oder deren Salzen, wie sie nach einem der Ansprüche 1 bis 5 und 7 definiert sind, als herbizide Wirkstoffe zur Bekämpfung von Schadpflanzen in Reiskulturen.

## Claims

1. A method of controlling harmful plants in rice crops, which comprises applying one or more compounds of the formula (I) or salts thereof 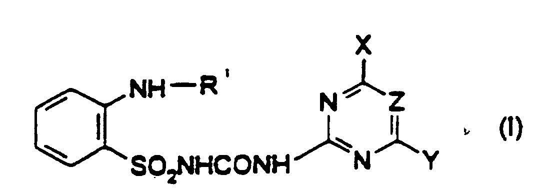 where
R¹ is an acyl radical from the group CO-R², CS-R², CO-OR³, CS-OR³, CO-SR⁴, CS-SR⁴, CO-NR⁵R⁶ and CS-NR⁵R⁶,
R² is H, (C₁-C₆)alkyl, (C₃-C₆)cycloalkyl, (C₃-C₆)cycloalkyl-(C₁-C₄)alkyl, (C₂-C₆)alkenyl or (C₂-C₆)alkynyl, where each of the 5 last-mentioned radicals is unsubstituted or substituted by one or more radicals from the group halogen, (C₁-C₄)alkoxy, (C₁-C₄)haloalkoxy, (C₁-C₄)alkylthio, (C₁-C₄)haloalkylthio, CN, NH₂, mono- and di(C₁-C₄)alkylamino and, in the case of cyclic radicals, also by (C₁-C₄)alkyl and (C₁-C₄)haloalkyl,
R³ is a radical from the group of the radicals which are possible for R², except for H,
R⁴ is a radical from the group of the radicals which are possible for R³,
R⁵,R⁶ independently of one another are each a radical from the group of the radicals which are possible for R², or the group
NR⁵R⁶ together is a substituted or unsubstituted 4-, 5- or 6-membered saturated or unsaturated heterocyclic radical which is bonded at the nitrogen atom, which, in addition to the nitrogen atom, contains no further hetero ring atom or further hetero ring atoms and which is unsubstituted or substituted by one or more radicals from the group (C₁-C₄)alkyl, (C₁-C₄)haloalkyl, halogen, (C₁-C₄)alkoxy and (C₁-C₄)haloalkoxy,
X,Y independently of one another are halogen, (C₁-C₆)alkyl, (C₁-C₆)alkoxy, (C₁-C₆)alkylthio, where each of the three last-mentioned radicals is unsubstituted or substituted by one or more radicals from the group halogen, (C₁-C₄)alkoxy and (C₁-C₄)alkylthio, or is (C₃-C₆)cycloalkyl, (C₃-C₆)alkenyl, (C₃-C₆)-alkynyl, (C₃-C₆)alkenyloxy or (C₃-C₆)alkynyloxy and
Z is CH or N,
to the area under cultivation, which contains the harmful plants and rice plants or their seeds, in an effective amount of from 0.0001 to 0.5 kg of a.i./ha.

2. The method as claimed in claim 1, wherein
R¹ is CO-R², CS-R², CO-OR³, CS-OR³, CO-SR⁴, CS-SR⁴, CO-NR⁵R⁶ and CS-NR⁵R⁶,
R² is H, (C₁-C₆)alkyl, (C₃-C₆)cycloalkyl, (C₃-C₆)cycloalkyl-(C₁-C₂)alkyl, (C₂-C₄)alkenyl or (C₂-C₄)alkynyl, where each of the 5 last-mentioned radicals is unsubstituted or substituted by one or more radicals from the group halogen, (C₁-C₃)alkoxy, (C₁-C₃)haloalkoxy, (C₁-C₃)alkylthio, (C₁-C₃)haloalkylthio, CN, NH₂, mono- and di(C₁-C₃)alkylamino and, in the case of cyclic radicals, also by (C₁-C₄)alkyl and (C₁-C₄)haloalkyl,
R³ is a radical of the group of the radicals which are possible for R², except for H,
R⁴ is a radical from the group of the radicals which are possible for R³,
R⁵,R⁶ independently of one another are each a radical from the group of the radicals which are possible for R², or the group
NR⁵R⁶ together is a substituted or unsubstituted 4-, 5- or 6-membered saturated heterocyclic radical of the formula (A-1), (A-2), (A-3) or (A-4) 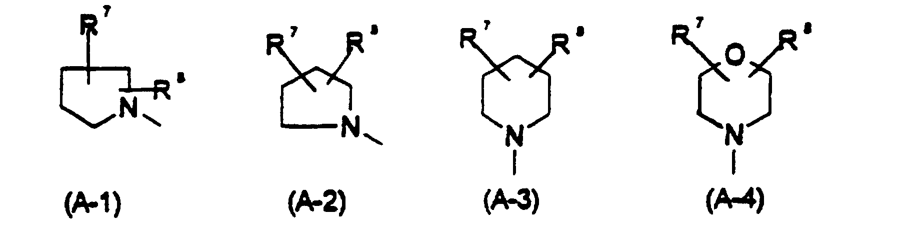 which is bonded at the nitrogen atom,
R⁷, R⁸ independently of one another are H, halogen, (C₁-C₃)alkyl, (C₁-C₃)haloalkyl, (C₁-C₃)alkoxy or (C₁-C₃)haloalkoxy,
X, Y independently of one another are halogen, (C₁-C₄)alkyl, (C₁-C₄)alkoxy, (C₁-C₄)alkylthio, where each of the three last-mentioned radicals is unsubstituted or substituted by one or more radicals from the group halogen, (C₁-C₄)alkoxy and (C₁-C₄)alkylthio, or is (C₃-C₆)cycloalkyl, (C₃-C₄)alkenyl, (C₃-C₄)-alkynyl, (C₃-C₄)alkeneoxy or (C₃-C₄)alkyneoxy and
Z is CH or N.

3. The method as claimed in claim 1 or 2, wherein
R² is H, (C₁-C₄)alkyl, (C₁-C₄)haloalkyl, (C₁-C₃)-alkoxy-(C₁-C₄)alkyl, (C₁-C₃)alkylthio-(C₁-C₄)-alkyl, (C₃-C₆)cycloalkyl or (C₃-C₆)cycloalkylmethyl,
R³ is (C₁-C₄)alkyl, (C₁-C₄)haloalkyl, (C₁-C₃)-alkoxy-(C₁-C₄)alkyl, (C₁-C₃)alkylthio- (C₁-C₄)-alkyl, (C₃-C₆)cycloalkyl, (C₃-C₆)cycloalkylmethyl, (C₃-C₄)alkenyl or (C₃-C₄)alkynyl,
R⁴ is a radical from the group of the radicals which are possible for R³,
R⁵,R⁶ independently of one another are H or (C₁-C₄)-alkyl, one of the radicals X and Y is (C₁-C₄)-alkyl, (C₁-C₄)alkoxy, (C₁-C₄)haloalkyl or (C₁-C₄)haloalkoxy,
and the other of the radicals X and Y is halogen, (C₁-C₄)alkyl, (C₁-C₄)alkoxy, (C₁-C₄)alkylthio, where the three last-mentioned radicals are unsubstituted or substituted by one or more radicals from the group halogen, (C₁-C₄)alkoxy and (C₁-C₄)alkylthio, or is (C₃-C₆)cycloalkyl, (C₂-C₄)alkenyl, (C₂-C₄)alkynyl, (C₃-C₄)alkeneoxy or (C₃-C₄)alkyneoxy and
Z is CH or N.

4. The method as claimed in any of claims 1 to 3, wherein
R¹ is COR² or CO-OR³,
R² is H, (C₁-C₄)alkyl or (C₃-C₄)cycloalkyl,
R³ is (C₁-C₄)alkyl or (C₃-C₆)cycloalkyl,
one of the radicals X and Y is methoxy, ethoxy or methyl and
the other of the radicals X and Y is methoxy, ethoxy, methyl, chlorine, fluorine or CF₃ and
Z is CH or N.

5. The method as claimed in claim 4, wherein
R² is ethyl, isopropyl or cyclopropyl,
R³ is methyl or ethyl,
X,Y are each methoxy and
Z is CH.

6. The method as claimed in any of claims 1 to 5, wherein the compounds (I) or salts thereof are applied at an application rate of 0.001 to 0.2 kg of a.i./ha.

7. A salt of the formula (Ia) 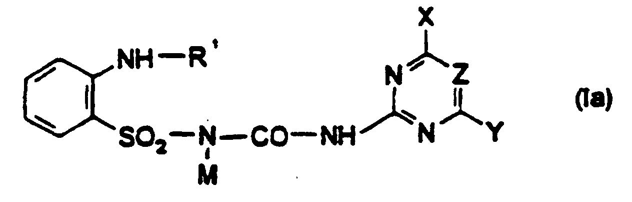 where
M is the equivalent of a cation and
R¹, R², R³, R⁴, X and Y are as defined in the formula (I) as claimed in any of claims 1 to 5.

8. A process for preparing the salt as claimed in claim 7, which comprises reacting the compounds of the formula (Ia) where M = H with bases.

9. The use of compounds of the formula (I) or (Ia) or salts thereof as defined by any of claims 1 to 5 and 7 as herbicidally active compounds for controlling harmful plants in rice crops.

## Revendications

1. Procédé pour lutter contre des plantes nuisibles dans des cultures de riz, **caractérisé en ce qu'**on applique sur la surface cultivable avec des plants de riz ou des plantes adventices ou leurs semences un ou plusieurs composés de formule (I) ou leurs sels 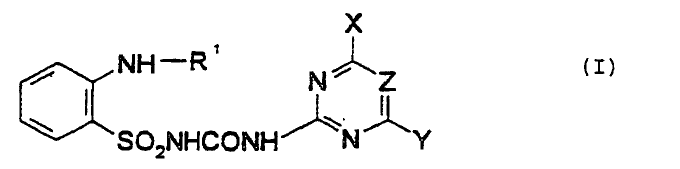 où
R¹ représente un reste acyle pris dans le groupe comportant CO-R², CS-R², CO-OR³, CS-OR³, CO-SR⁴, CS-SR⁴, CO-NR⁵R⁶ et CS-NR⁵R⁶,
R² représente un atome d'hydrogène, des restes alkyle en C₁-C₆, cycloalkyle en C₃-C₆, (cycloalkyle en C₃-C₆)-alkyle en C₁-C₄, alcényle en C₂-C₆ ou alcynyle en C₂-C₆, chacun des 5 derniers restes cités est non substitués ou substitués par un ou plusieurs restes pris dans le groupe comportant halogène, alkoxy en C₁-C₄, haloalkoxy en C₁-C₄, (alkyl en C₁-C₄)thio, halo(alkyl en C₁-C₄)thio, CN, NH₂, mono- ou di(alkyl en C₁-C₄)amino et dans le cas de reste cycliques, également par alkyle en C₁-C₄ et haloalkyle en C₁-C₄,
R³ représente un reste pris dans le groupe des restes possibles pour R² à l'exception de H,
R⁴ représente un reste pris dans le groupe des restes possibles pour R³,
R⁵, R⁶ représentent chacun, indépendamment l'un de l'autre, un reste pris dans le groupe des restes possibles pour R² ou le groupe
NR⁵R⁶ ensemble représentent un reste hétérocyclique saturé ou insaturé, à 4, 5 ou 6 chaînons, substitué ou non substitué, lié à l'atome de N, lequel ne présente pas d'autres hétéroatomes au côté de l'atome de N ou contient d'autres hétéroatomes dans le cycle et qui est non substitué ou substitué par un ou plusieurs restes pris dans le groupe comportant alkyle en C₁-C₄, haloalkyle en C₁-C₄, halogène, alkoxy en C₁-C₄ et haloalkoxy en C₁-C₄,
X, Y représentent, indépendamment l'un de l'autre, des atomes d'halogène, des restes alkyle en C₁-C₆, alkoxy en C₁-C₆, (alkyl en C₁-C₆)thio, chacun des trois derniers restes cités pouvant être non substitué ou substitué par un ou plusieurs restes pris dans le groupe comportant halogène, alkoxy en C₁-C₄ et (alkyl en C₁-C₄)-thio, ou représente cycloalkyle en C₃-C₆, alcényle en C₃-C₆, alcynyle en C₃-C₆, (alcényl en C₃-C₆)oxy ou (alcynyl en C₃-C₆)oxy et
Z représente CH ou N,
en une quantité efficace de 0,0001 à 0,5 kg i.a./ha.

2. Procédé selon la revendication 1, **caractérisé en ce que**
R¹ représente CO-R², CS-R², CO-OR³, CS-OR³, CO-SR⁴, CS-SR⁴, CO-NR⁵R⁶ et CS-NR⁵CR⁶,
R² représente un atome d'hydrogène, des restes alkyle en C₁-C₆, cycloalkyle en C₃-C₆, (cycloalkyle en C₃-C₆)-alkyle en C₁-C₂, alcényle en C₂-C₄ ou alcynyle en C₂-C₄, chacun des 5 derniers restes cités étant non substitué ou substitué par un ou plusieurs restes pris dans le groupe comportant halogène, alkoxy en C₁-C₃, haloalkoxy en C₁-C₃, (alkyl en C₁-C₃)thio, halo(alkyl en C₁-C₃)thio, CN, NH₂, mono- ou di(alkyl en C₁-C₃)amino et dans le cas de restes cycliques, également par alkyle en C₁-C₄ et haloalkyle en C₁-C₄,
R³ représente un reste pris dans le groupe des restes possibles pour R² à l'exception de H,
R⁴ représente un reste pris dans le groupe des restes possibles pour R³,
R⁵, R⁶ représentent chacun, indépendamment l'un de l'autre, un reste pris dans le groupe des restes possibles pour R² ou le groupe
NR⁵R⁶ ensemble représentent un reste hétérocyclique saturé, à 4, 5 ou 6 chaînons, substitué ou non substitué, lié à l'atome de N, de formule (A-1), (A-2), (A-3) ou (A-4) 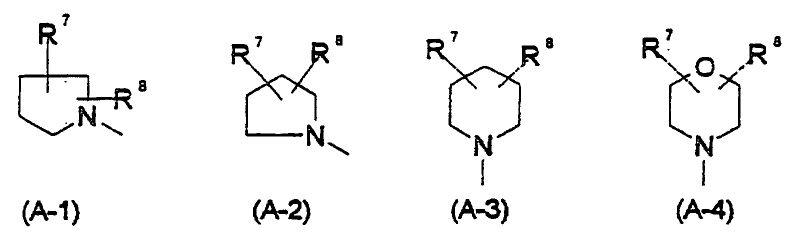
R⁷, R⁸ représentent indépendamment l'un de l'autre, un atome d'hydrogène, des restes halogène, alkyle en C₁-C₃, haloalkyle en C₁-C₃, alkoxy en C₁-C₃ ou haloalkoxy en C₁-C₃,
X, Y représentent, indépendamment l'un de l'autre, des atomes d'halogène, des restes alkyle en C₁-C₄, alkoxy en C₁-C₄, (alkyl en C₁-C₄)thio, chacun des trois derniers restes cités pouvant être non substitué ou substitué par un ou plusieurs restes pris dans le groupe comportant halogène, alkoxy en C₁-C₄ et (alkyl en C₁-C₄)thio, ou représente cycloalkyle en C₃-C₆, alcényle en C₃-C₄, alcynyle en C₃-C₄, (alcényl en C₃-C₄)oxy ou (alcynyl en C₃-C₄)oxy et
Z représente CH ou N.

3. Procédé selon la revendication 1 ou 2, **caractérisé en ce que**
R² représente un atome d'hydrogène, des restes alkyle en C₁-C₄, haloalkyle en C₁-C₄, (alkoxy en C₁-C₃)-alkyle en C₁-C₄, (alkyl en C₁-C₃)thioalkyle en C₁-C₄, cycloalkyle en C₃-C₆ ou (cycloalkyl en C₃-C₆)méthyle,
R³ représente des restes alkyle en C₁-C₄, haloalkyle en C₁-C₄, (alkoxy en C₁-C₃)-alkyle en C₁-C₄, (alkyl en C₁-C₃)thio-alkyle en C₁-C₄, cycloalkyle en C₃-C₆, (cycloalkyl en C₃-C₆)-méthyle, alcényle en C₃-C₄ ou alcynyle en C₃-C₄,
R⁴ représente un reste pris dans le groupe des restes possibles pour R³,
R⁵, R⁶ représentent chacun, indépendamment l'un de l'autre, un atome d'hydrogène ou l'un reste alkyle en C₁-C₄, un des restes X et Y représente des groupes alkyle en C₁-C₄, alkoxy en C₁-C₄, haloalkyle en C₁-C₄ ou haloalkoxy en C₁-C₄,
et l'autre des restes X et Y représente des atomes d'halogène, des restes alkyle en C₁-C₄, alkoxy en C₁-C₄, (alkyl en C₁-C₄)thio, chacun des trois derniers restes cités pouvant être non substitué ou substitué par un ou plusieurs restes pris dans le groupe comportant halogène, alkoxy en C₁C₄ et (alkyl en C₁-C₄)thio, ou représente cycloalkyle en C₃-C₆, alcényle en C₂-C₄, alcynyle en C₂-C₄, (alcényl en C₃-C₄)oxy ou (alcynyl en C₃-C₄)oxy et
Z représente CH ou N.

4. Procédé selon l'une des revendications 1 à 3, **caractérisé en ce que**
R¹ représente un reste CO-R² ou CO-OR³,
R² représente un atome d'hydrogène, un reste alkyle en C₁-C₄ ou cycloalkyle en C₃-C₄,
R³ représente un reste alkyle en C₁-C₄ ou cycloalkyle en C₃-C₆,
l'un des restes X et Y représente un groupe méthoxy, éthoxy ou méthyle et
l'autre des restes X et Y représente des groupes méthoxy, éthoxy, méthyle, chloro, fluoro ou CF₃ et
Z représente des groupes CH ou N.

5. Procédé selon la revendication 4, **caractérisé en ce que**
R² représente un reste éthyle, isopropyle ou cyclopropyle,
R³ représente un reste méthyle ou éthyle,
X et Y représentent chacun un reste méthoxy et
Z représente un groupe CH.

6. Procédé selon l'une des revendications 1 à 5, **caractérisé en ce qu'**on applique le composé (I) ou ses sels en une dose de 0,001 à 0,2 kg i.a./ha.

7. Sels de formule (Ia) 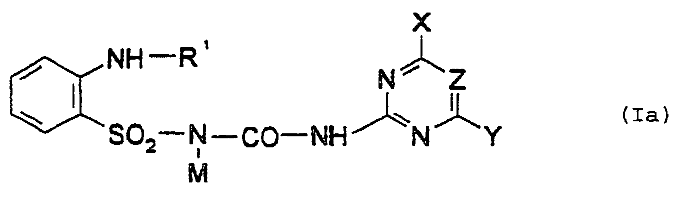 où
M représente l'équivalent d'un cation et
R¹, R², R³, R⁴, X et Y sont définis comme à la formule (I) selon l'une des revendications 1 à 5.

8. Procédé pour la préparation des sels selon la revendication 7, **caractérisé en ce qu'**on fait réagir les composés de formule (Ia), où M = H, avec des bases.

9. Utilisation de composés de formules (I) ou (Ia) ou de leurs sels, tels que définis selon l'une des revendications 1 à 5 et 7 comme substances actives herbicides pour la lutte contre les plantes nuisibles dans des cultures de riz.
